# EUROPEAN PATENT APPLICATION

(11) **EP 2 248 750 A1**
(43) Date of publication of application: **10.11.2010**
(21) Application number: 09716769.6
(22) Date of filing: 04.02.2009
(51) Int. Cl.: B65H 23/192, A61F 13/15, A61F 13/49, B65H 20/04, B65H 20/24

(54) **PROCESSING APPARATUS**

(30) Priority: 06.03.2008 JP 2008056618
(71) Applicant: Uni-Charm Corporation, Ehime 799-0111 (JP)
(72) Inventor: KAMEDA, Noritomo, Kanonji-shi Kagawa 769-1602 (JP)
(74) Representative: Fitchett, Stuart Paul
(86) International application number: PCT/JP2009/051885
(87) International publication number: WO 2009/110278

(57) **Abstract**

In a processing apparatus (10) that intermittently halts and performs processing (Pn) on a continuous belt-like work (1) while causing the work (1) to travel along a predetermined travel path (PL), a variation in tension of the work (1) that may arise in association with the intermittent halting of the work (1) is effectively suppressed.

A processing apparatus (10) includes: a work carrying section (30) that carries a continuous belt-like work (1) by wrapping the work (1) around a plurality of carry rolls (31, 32) and forming a travel path (PL) for the work (1); and a processing section (20) that is provided within the travel path (PL) and that performs processing while the work (1) is being intermittently halted. The work carrying section (30) includes an entering-side buffer mechanism (34b) that is provided within the travel path (PL) on an upstream side of the processing section (20) and that can accumulate the work (1) carried from upstream by wrapping the work around a first moving element (34a) that is guided to be able to perform a reciprocating movement, an exit-side buffer mechanism (34b) that is provided within the travel path (PL) on a downstream side of the processing section (20) and that can accumulate the work (1) having been processed and to be carried downstream by wrapping the work having been processed around a second moving element (34b) that is guided to be able to perform a reciprocating movement, an interlocking section (34) that moves the first moving element (34a) and the second moving element (34b) in an interlocked manner in such a manner that a motion of the entering-side buffer mechanism (34a) for increasing or decreasing an accumulation amount of the work (1) accumulated thereby and a motion of the exit-side buffer mechanism (34b) for increasing or decreasing an accumulation amount of the work (1) accumulated thereby are opposite from one another and in such a manner that an amount of change in the accumulation amount of the work (1) of the entering-side buffer mechanism (34a) and an amount of change in the accumulation amount of the work (1) of the exit-side buffer mechanism (34b) are equal, an intermittent sending section (31b) that comes into contact with the work (1) which has passed through the entering-side buffer mechanism (34a) and that repeats a halt motion for intermittently halting the work (1) and a sending motion for sending out the work to the processing section (20), an endless annular element (37a) that is wrapped around the carry rolls (31, 32), the first moving element (34a), and the second moving element (34b) in such a manner that the endless annular element lies along the work (1) within the travel path (PL), and a first drive roll (37e) that, by circulatingly driving the endless annular element (37a), applies a rotational force to the carry rolls (31, 32) and also applies a moving force to the first moving element (34a) and the second moving element (34b) for achieving the reciprocating movement thereof. The first drive roll (37e) continuously drives the endless annular element (37a) circulatingly at a predetermined speed while the intermittent sending section (31b) intermittently halts the endless annular element (37a) in synchronization with the halt motion of the work (1) in such a manner that the endless annular element (37a) is subjected to the same travel motion and accumulation motion as those of the work (1) within a range where the endless annular element (37a) lies along the travel path (PL).

## Description

### Technical Field

The present invention relates to a processing apparatus for intermittently halting and processing a continuous belt-like work while causing the work to travel along a predetermined travel path.

### Background Art

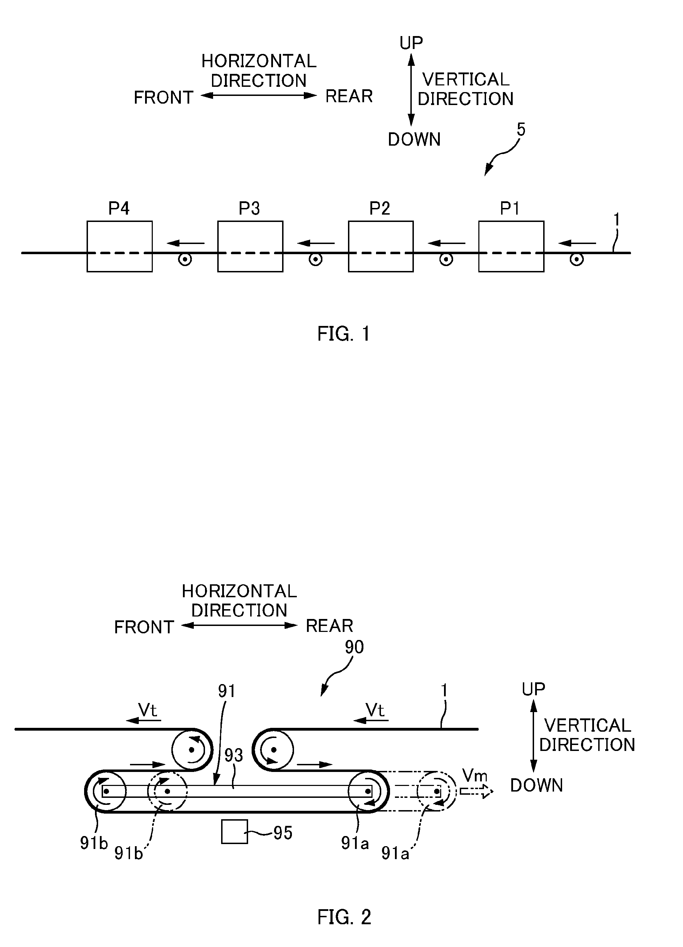

As shown in Fig. 1, a conventional production line 5 for disposable diapers, for example, includes a plurality of processing positions P1, P2,... aligned front-to-rear along the horizontal direction. Semi-finished diapers are carried continuously in the form of a continuous belt-like work 1, and during this course, various processes are applied thereto at the respective processing positions P1, P2,... to thereby complete finished products.

In cases where press-working is to be applied at one of the above-mentioned processing positions P1, P2,..., carrying of the belt-like work 1 will be intermittently halted with every die-pressing motion using press dies. This halt, however, affects the carrying at processing positions immediately upstream and downstream of the press-working position. That is, it is inevitable that the belt-like work 1 will temporarily be halted also at the immediately adjacent processing positions.

In this regard, a processing apparatus 90 disclosed in Patent Literature 1 allows the carrying of the belt-like work 1 to be intermittently halted only at the processing apparatus 90, without stopping the carrying thereof at positions upstream and downstream of the processing apparatus 90.

More specifically, as shown in Fig. 2, the processing apparatus 90 includes a roll pair 91 consisting of a pair of rolls 91a, 91b connected together while being spaced-away from one another in the front and rear direction by a separator 93. A belt-like work 1 is wrapped around the roll pair 91, thus forming a travel path of the belt-like work 1. Moving the roll pair 91 at the same speed as the travel speed Vt of the belt-like work 1 in the opposite direction therefrom allows the moving speed Vm and the travel speed Vt to cancel out one another. This creates a pseudo-halted state of the belt-like work 1 at a position between the rolls 91a, 91b, and during this pseudo-halted state, processing is applied by a processing section 95.
Patent Literature 1: Japanese Patent No. 3452577

### Disclosure of Invention

### Problem to be solved by the Invention

The above-described halted state, however, is not one that halts the belt-like work 1 physically. Therefore, there are cases where the belt-like work 1 may slightly shift owing to synchronization failure between the moving speed Vm and the travel speed Vt, for example. Thus, the position for processing the belt-like work 1 may deviate from the target position.

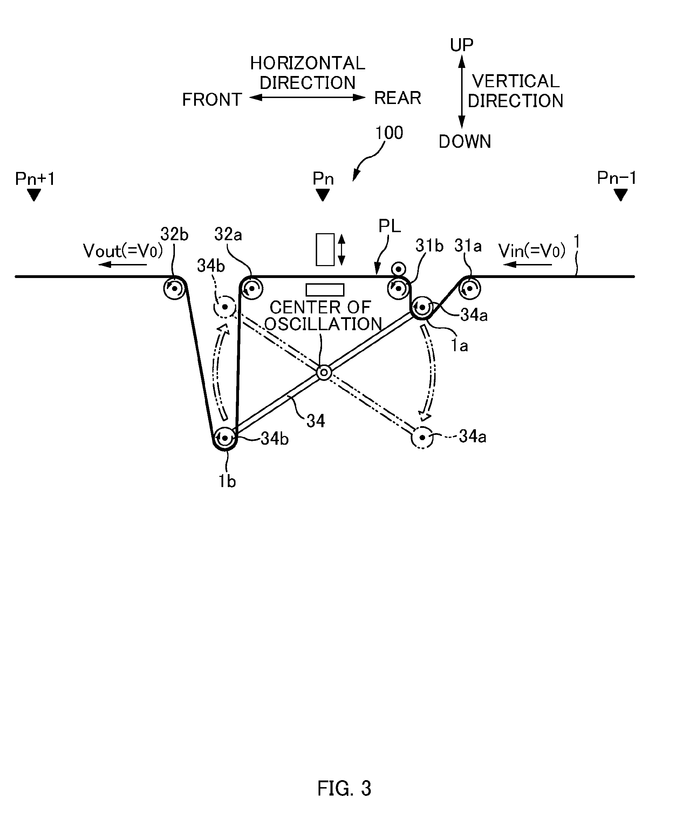

In this regard, an apparatus 100 such as the one shown in the side view of Fig. 3 may be contemplated as a processing apparatus for solving the above problem. This processing apparatus 100 includes a plurality of carry rolls 31a, 31b, 32a, 32b that form a travel path PL of the belt-like work 1, and a seesaw member 34 arranged so as to extend front-to-rear across a processing position Pn set within the travel path PL. The belt-like work 1 is wrapped around rolls 34a, 34b provided at the respective front and rear ends of the seesaw member 34. The oscillating motion of the seesaw member 34 makes it possible to form loops 1a, 1b consisting of the belt-like work 1 in the front and rear of the processing position Pn and thereby accumulate the belt-like work 1. A carry roll 31b located between the processing position Pn and the roll 34a of the seesaw member 34 is a drive roll 31b that is driven to rotate by a servomotor, for example. This drive roll 31b contacts the belt-like work 1.

Accordingly, with this processing apparatus 100, it is possible to reliably achieve a physically-halted state of the belt-like work 1 at the processing position Pn by halting the drive roll 31b.

During this halt, the belt-like work 1 sent in from an upstream processing position Pn-1 to the processing apparatus 100 is accumulated as a result of the upstream loop 1a becoming large due to the seesawing motion of the seesaw member 34, which is shown by the chain double-dashed lines of Fig. 3, whereas the processed belt-like work 1 that is to be sent out from the processing apparatus 100 to a downstream processing position Pn+1 is forwarded as a result of the downstream loop 1b becoming small due to the same seesawing motion. In this way, the influence of the halting of the belt-like work 1 at the processing apparatus 100 is kept from spilling over to the immediately upstream and downstream processing positions Pn-1, Pn+1.

The seesaw member 34, however, is a driven (following) component that oscillates up and down by receiving a force from the belt-like work 1. The carry roll 32a is also a driven roll that rotates by receiving a rotational force from the belt-like work 1. Therefore, when the belt-like work 1 is halted, the roll 34b of the seesaw member 34 is pulled up by the belt-like work 1 and starts to ascend. However, at this time, inertia of the oscillating motion of the seesaw member 34, for example, acts on the belt-like work 1, and this causes a significant variation in tension applied to the belt-like work 1. Also, upon halt, the carry roll 32a having been rotating following the belt-like work 1 is halted. Therefore, inertia of the rotating motion of the carry roll 32a acts on the belt-like work 1, and this also causes a variation in tension applied to the belt-like work 1.

The present invention has been made in view of such a conventional problem as that described above, and an object thereof is to provide a processing apparatus that intermittently halts and processes a continuous belt-like work while causing the work to travel along a predetermined travel path, and that can effectively suppress a variation in tension of the work that may arise in association with the intermittent halting of the work.

### Means for Solving the Problem

A main aspect of the invention for achieving the above-mentioned object is a processing apparatus having:
a work carrying section that carries a continuous belt-like work by wrapping the work around a plurality of carry rolls and forming a travel path for the work; and
a processing section that is provided within the travel path and that performs processing while the work is being intermittently halted;
   wherein the work carrying section includes
   an entering-side buffer mechanism that is provided within the travel path on an upstream side of the processing section and that can accumulate the work carried from upstream by wrapping the work around a first moving element that is guided to be able to perform a reciprocating movement,
   an exit-side buffer mechanism that is provided within the travel path on a downstream side of the processing section and that can accumulate the work having been processed and to be carried downstream by wrapping the work having been processed around a second moving element that is guided to be able to perform a reciprocating movement,
   an interlocking section that moves the first moving element and the second moving element in an interlocked manner in such a manner that a motion of the entering-side buffer mechanism for increasing or decreasing an accumulation amount of the work accumulated thereby and a motion of the exit-side buffer mechanism for increasing or decreasing an accumulation amount of the work accumulated thereby are opposite from one another and in such a manner that an amount of change in the accumulation amount of the work of the entering-side buffer mechanism and an amount of change in the accumulation amount of the work of the exit-side buffer mechanism are equal,
   an intermittent sending section that comes into contact with the work which has passed through the entering-side buffer mechanism and that repeats a halt motion for intermittently halting the work and a sending motion for sending out the work to the processing section,
   an endless annular element that is wrapped around the carry rolls, the first moving element, and the second moving element in such a manner that the endless annular element lies along the work within the travel path, and
   a first drive roll that, by circulatingly driving the endless annular element, applies a rotational force to the carry rolls and also applies a moving force to the first moving element and the second moving element for achieving the reciprocating movement thereof; and
   wherein the first drive roll continuously drives the endless annular element circulatingly at a predetermined speed while the intermittent sending section intermittently halts the endless annular element in synchronization with the halt motion of the work in such a manner that the endless annular element is subjected to the same travel motion and accumulation motion as those of the work within a range where the endless annular element lies along the travel path.
   Other features of the invention will be made clear by the disclosure of the present description and accompanying drawings.

### Effect of the Invention

The present invention can provide a processing apparatus that intermittently halts and processes a continuous belt-like work while causing the work to travel along a predetermined travel path, and that can effectively suppress a variation in tension of the work that may arise in association with the intermittent halting of the work.

### Brief Description of Drawings

Fig. 1 is a conceptual diagram of a production line 5 having a plurality of processing positions P1, P2,....
Fig. 2 is a side view showing an example of a conventional processing apparatus 90.
Fig. 3 is a side view of a processing apparatus 100 that solves the problem of the conventional processing apparatus 90.
Figs. 4A and 4B are side views of a processing apparatus 10 according to a first embodiment.
Figs. 5A to 5C are explanatory diagrams describing an oscillating motion of a seesaw member 34 during the intermittent halt of a belt-like work 1.
Figs. 6A to 6C are explanatory diagrams describing an oscillating motion of the seesaw member 34 when the intermittent halt of the belt-like work 1 is released.
Figs. 7A to 7C are explanatory diagrams describing an oscillating motion of the seesaw member 34 during the intermittent halt of the belt-like work 1.
Figs. 8A to 8C are explanatory diagrams describing an oscillating motion of the seesaw member 34 when the intermittent halt of the belt-like work 1 is released.
Fig. 9 is a chart indicating a rotation speed V of a drive roll 31b. Fig. 10 is a side view of a processing apparatus 10a according to a second embodiment.
Fig. 11 is a side view of a processing apparatus 10b according to a modified example of the second embodiment.
Fig. 12 is a side view of a processing apparatus 10c according to another embodiment.
Fig. 13 is a side view of a processing apparatus 10d according to another embodiment.

### List of Reference Numerals

1: belt-like work (work); 1a: portion (loop); 1b: portion (loop);
5: continuous production line; 10: processing apparatus;
10a: processing apparatus; 10c: processing apparatus;
10d: processing apparatus; 20: press device (processing section);
21a: male die; 21b: female die;
30: intermittent carry device (work carrying section);
31: group of pass-line rolls (carry rolls);
31a: pass-line roll (carry roll);
31b: drive roll (intermittent sending section, second drive roll);
31c: pressing roll;
31d: brake-equipped non-drive roll (intermittent sending section);
32: group of pass-line rolls (carry rolls);
32a: pass-line roll (carry roll); 32b: pass-line roll (carry roll);
34: seesaw member (interlocking section);
34a: entering-side roll (first moving element, entering-side buffer mechanism);
34b: exit-side roll (second moving element, exit-side buffer mechanism) ;
34c: oscillation central shaft;
36: air cylinder (intermittent sending section, oscillation drive device);
36a: piston; 37: tension-variation-suppressing mechanism;
37a: endless belt (endless annular element); 37b: driven roll;
37c: driven roll; 37d: driven roll; 37e: drive roll (first drive roll);
37f: driven roll; 37g: driven roll; 38: plate cam (intermittent sending section, oscillation drive device); 38c: axis;
41: pressing-motion monitoring sensor;
42: processing-target-section monitoring sensor;
43: oscillating-motion monitoring sensor;
43a: sensor at upper lower-limit position;
43b: sensor at lower lower-limit position;
90: processing apparatus; 91a: roll; 91b: roll; 93: separator;
95: processing section; 100: processing apparatus; 1La: loop; 1Lb: loop; Pn-1: processing position; Pn: processing position;
Pn+1: processing position; PL: pass line (travel path);
V: carry speed (travel speed);
Vin: entering-side carry speed (travel speed);
Vout: exit-side carry speed (travel speed);
A1: normal range; A2: deceleration range; A3: intermittent halt range; A4: acceleration range; A5: deceleration range; Vt: travel speed;
Vm: moving speed

### Best Mode for Carrying Out the Invention

At least the following matters will be made clear by the disclosure of the present description and the accompanying drawings.

A processing apparatus includes:
a work carrying section that carries a continuous belt-like work by wrapping the work around a plurality of carry rolls and forming a travel path for the work; and
a processing section that is provided within the travel path and that performs processing while the work is being intermittently halted;
   wherein the work carrying section includes
   an entering-side buffer mechanism that is provided within the travel path on an upstream side of the processing section and that can accumulate the work carried from upstream by wrapping the work around a first moving element that is guided to be able to perform a reciprocating movement,
   an exit-side buffer mechanism that is provided within the travel path on a downstream side of the processing section and that can accumulate the work having been processed and to be carried downstream by wrapping the work having been processed around a second moving element that is guided to be able to perform a reciprocating movement,
   an interlocking section that moves the first moving element and the second moving element in an interlocked manner in such a manner that a motion of the entering-side buffer mechanism for increasing or decreasing an accumulation amount of the work accumulated thereby and a motion of the exit-side buffer mechanism for increasing or decreasing an accumulation amount of the work accumulated thereby are opposite from one another and in such a manner that an amount of change in the accumulation amount of the work of the entering-side buffer mechanism and an amount of change in the accumulation amount of the work of the exit-side buffer mechanism are equal,
   an intermittent sending section that comes into contact with the work which has passed through the entering-side buffer mechanism and that repeats a halt motion for intermittently halting the work and a sending motion for sending out the work to the processing section,
   an endless annular element that is wrapped around the carry rolls, the first moving element, and the second moving element in such a manner that the endless annular element lies along the work within the travel path, and
   a first drive roll that, by circulatingly driving the endless annular element, applies a rotational force to the carry rolls and also applies a moving force to the first moving element and the second moving element for achieving the reciprocating movement thereof; and
   wherein the first drive roll continuously drives the endless annular element circulatingly at a predetermined speed while the intermittent sending section intermittently halts the endless annular element in synchronization with the halt motion of the work in such a manner that the endless annular element is subjected to the same travel motion and accumulation motion as those of the work within a range where the endless annular element lies along the travel path.

With such a processing apparatus, a rotational force is applied to the carry rolls and a moving force is applied to the first moving element and the second moving element for achieving the reciprocating movement thereof by circulatingly driving the endless annular element. This reduces the action, on the work, of inertia relating to the rotating motion of the carry rolls as well as inertia relating to the movement motion of the first moving element and the second moving element. Thus, it is possible to suppress a variation in tension of the work.

Further, the first drive roll and the intermittent sending section allow the endless annular element to be subjected to the same travel motion and accumulation motion as those of the work within a range where the endless annular element lies along the work. Therefore, the endless annular element can reliably receive inertia relating to the rotating motion of the carry rolls as well as inertia relating to the movement motion of the first and second moving elements, which may act on the work at arbitrary timings, in place of the work. Thus, it is possible to effectively suppress a variation in tension of the work.

Note that when the intermittent sending section halts the work at the processing section for the processing apparatus to perform processing, the work that is sent in from the upstream of the processing apparatus is accumulated by the entering-side buffer mechanism, whereas the processed work that has been accumulated is sent out from the exit-side buffer mechanism to the downstream of the processing apparatus. Accordingly, the influence of halting the work at the processing apparatus is effectively kept from spilling over to the carrying of the work at the immediately upstream and downstream processing positions.

Further, since the intermittent sending section comes into contact with the work to halt the work, the work is braked physically. The processing can thus be applied to the position on the work targeted for processing with high accuracy.

Further, by providing the interlocking section, the increase or decrease motions for increasing or decreasing the accumulation amount of the work are carried out in such a manner that the increase or decrease motions become opposite from one another between the entering-side buffer mechanism and the exit-side buffer mechanism and the amount of change in the accumulation amount of the work accumulated by each buffer mechanism is kept equal. Thus, it is possible to keep the tension of the work substantially constant both during halting of the work and during release of the halt.

In the above processing apparatus, it is preferable that: the first moving element of the entering-side buffer mechanism is a roll that rotates followingly; the second moving element of the exit-side buffer mechanism is a roll that rotates followingly; and the rolls rotate by receiving the rotational force caused by circulatingly driving the endless annular element.

With such a processing apparatus, the work wrapped around the first moving element and the second moving element is carried smoothly by the following rotation of the rolls serving as the first moving element and the second moving element.

Further, since the rolls rotate by receiving the rotational force caused by circulatingly driving the endless annular element, it is possible to effectively prevent inertia of the rotating motion of these rolls from acting on the work. Thus, it is possible to effectively suppress a variation in tension of the work.

In the above processing apparatus, it is preferable that: the processing apparatus further includes a seesaw member as the interlocking section, the seesaw member oscillating about a predetermined axis; the first moving element and the second moving element are mounted on the seesaw member; and the axis is located at a midpoint between a position where the first moving element is mounted and a position where the second moving element is mounted, and an oscillating motion of the seesaw member causes the reciprocating movement of the first moving element and the second moving element.

In this processing apparatus, a seesaw member is employed as the interlocking section. Thus, it is possible to easily achieve the state where the increase or decrease motions for increasing or decreasing the accumulation amount of the work are opposite from one another between the entering-side buffer mechanism and the exit-side buffer mechanism and the amount of change in the accumulation amount of the work accumulated by each buffer mechanism is kept equal.

In the above processing apparatus, it is preferable that the seesaw member includes a plurality of the first moving elements for wrapping the work around the first moving elements in a zigzag form, and a plurality of the second moving elements for wrapping the work having been processed around the second moving elements in a zigzag form.

With such a processing apparatus, the oscillation stroke amount of the seesaw member can be reduced, compared to a case where the work is not wrapped in a zigzag form, i.e., a case where one each of the first moving element and the second moving element is provided. Thus, it is possible to reduce the oscillating motion speed and thereby reduce the impact upon vertically reversing the oscillating motion.

In the above processing apparatus, it is preferable that: the predetermined speed of the endless annular element is a speed at a contacting position where the first drive roll comes into contact with the endless annular element; and the predetermined speed is equal to a travel speed of the work on a side upstream of the entering-side buffer mechanism or a travel speed of the work having been processed on a side downstream of the exit-side buffer mechanism.

With such a processing apparatus, the travel motion of the endless annular element can be made to match the travel motion of the work. Therefore, the endless annular element can reliably receive, in place of the work, inertia relating to the carry rolls as well as the first moving element and the second moving element, which may act on the work in association with the halt motion and/or the sending motion of the work. Thus, it is possible to effectively suppress a variation in tension of the work.

In the above processing apparatus, it is preferable that: the intermittent sending section is a second drive roll that carries the work to the processing section by being driven to rotate while winding the work from the entering-side buffer mechanism around an outer circumferential surface of the roll; and the endless annular element is also wound around the second drive roll.

With such a processing apparatus, the work is wound around the second drive roll and is carried due to the second drive roll being driven to rotate. Therefore, the work can reliably be braked by halting the second drive roll. Thus, it is possible to bring the work to a halted state reliably.

Further, since the endless annular element is also wound around the second drive roll serving as the intermittent sending section, it is possible to halt the endless annular element in synchronization with the halt motion of the work and also send out the endless annular element in synchronization with the sending motion of the work. That is, it is possible to enhance synchronism between the travel motion of the work and the travel motion of the endless annular element. Therefore, the endless annular element can reliably receive, in place of the work, inertia relating to the carry rolls as well as inertia relating to the first and second moving elements, which may act on the work in association with the halt motion and/or the sending motion. Thus, it is possible to effectively suppress a variation in tension of the work.

In the above processing apparatus, it is preferable that: while the halt motion of the work is cancelled, the intermittent sending section performs, in parallel, an increasing motion for increasing the accumulation amount of the exit-side buffer mechanism and a decreasing motion for decreasing the accumulation amount of the entering-side buffer mechanism; and the increasing motion and the decreasing motion are achieved by making a travel speed of the work between the entering-side buffer mechanism and the exit-side buffer mechanism faster than both a travel speed of the work on a side upstream of the entering-side buffer mechanism and a travel speed of the work having been processed on a side downstream of the exit-side buffer mechanism.

With such a processing apparatus, it is possible to reliably provide for the next work-halt motion. That is, the exit-side buffer mechanism can be returned to a state where it can send the work downstream by accumulating the processed work with the exit-side buffer mechanism, and the entering-side buffer mechanism can be returned to a state where it can accumulate the work sent in from upstream by sending out the work accumulated by the entering-side buffer mechanism.

### === FIRST EMBODIMENT ===

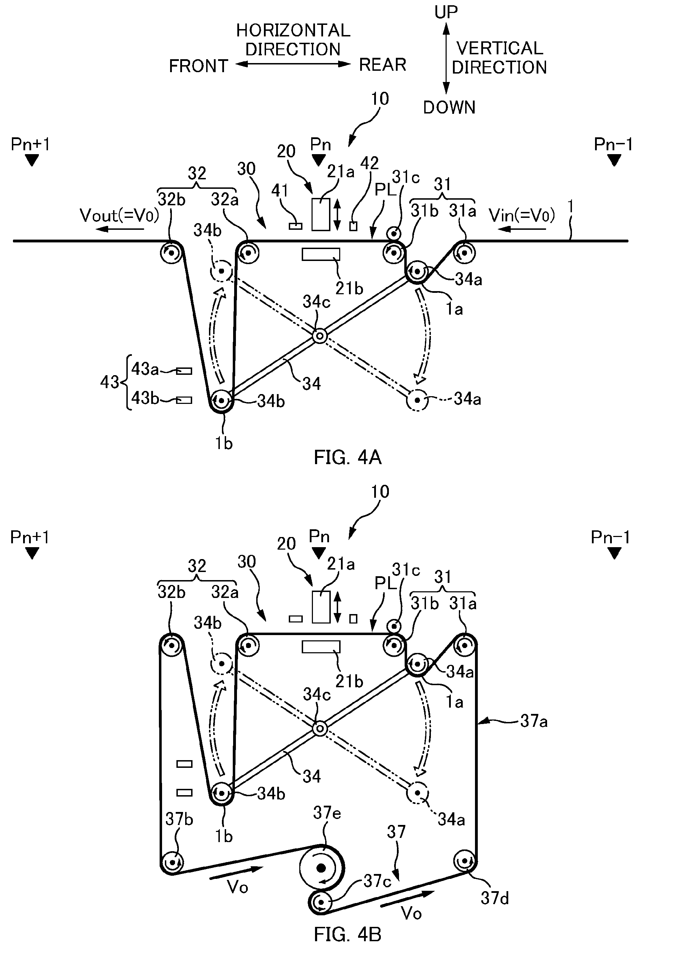

Figs. 4A and 4B are side views of a processing apparatus 10 according to a first embodiment. Note that, in order to keep the figures from becoming complicated, Fig. 4A shows the processing apparatus 10 without its tension-variation-suppressing mechanism 37, which is a component of the apparatus 10, and conversely, Fig. 4B shows the apparatus with its tension-variation-suppressing mechanism 37 but without the belt-like work 1.

Further, as shown in Fig. 4A, the vertical direction is referred to below also as the up and down direction, and the horizontal direction orthogonal to the vertical direction is referred to below also as the front and rear direction. Incidentally, the "front" of the front and rear direction is the downstream side of the carrying direction of the belt-like work 1, and the "rear" is the upstream side of the same. Further, the upstream side of the processing apparatus 10 is referred to also as the entering side, and the downstream side of the same is referred to also as the exit side.

The processing apparatus 10 according to the first embodiment is incorporated into one processing position Pn of the plurality of processing positions P1, P2,... provided in the continuous production line 5 of Fig. 1. As shown in Fig. 4A, the belt-like work 1 is drawn into the processing apparatus 10 from an immediately upstream processing position Pn-1 at an entering-side carry speed Vin, and on the other hand, after being subjected to predetermined processing by the processing apparatus 10, the work is drawn out toward an immediately downstream processing position Pn+1 at an exit-side carry speed Vout.

According to the constant mass flow principle, the entering-side carry speed Vin and the exit-side carry speed Vout of the processing apparatus 10 should basically be equal to one another; however, in cases where the belt-like work 1 is a stretchable material such as nonwoven fabric, the entering-side carry speed Vin and the exit-side carry speed Vout may differ from one another owing to the stretching deformation of the material, and for example, the entering-side carry speed Vin may range from 95% to 105% of the exit-side carry speed Vout. The explanation below, however, is based on the premise that the entering-side carry speed Vin and the exit-side carry speed Vout are controlled in such a manner that they are equal to one another and are at a reference speed V0.

Incidentally, the processing carried out by the processing apparatus 10 is, for example, press-working. It is therefore necessary to intermittently halt the carrying of the belt-like work 1 during this press-working. The processing apparatus 10, however, is devised so that it can keep the entering-side carry speed Vin and the exit-side carry speed Vout constant at approximately the above-mentioned reference speed V0, regardless of the intermittent halt. In other words, this processing apparatus 10 allows intermittent carrying to be performed only at the processing apparatus 10, while continuously carrying the belt-like work 1 at the immediately upstream and downstream processing positions Pn-1, Pn+1 without stopping the carrying thereat whatsoever. The following describes the processing apparatus 10 in detail.

As shown in Fig. 4A, the processing apparatus 10 includes: an intermittent carry device 30 serving as a work carrying section that carries a continuous belt-like work 1 by wrapping the belt-like work 1 around a plurality of carry rolls 31, 32 and forming a travel path PL for the belt-like work 1; a press device 20 serving as a processing section that is provided within the travel path PL and that performs processing while the belt-like work 1 is being intermittently halted; a group of sensors 41, 42, 43 for detecting the state of the press device 20 and the intermittent carry device 30; and a controller (not shown) for controlling the press device 20 and the intermittent carry device 30 in cooperation with one another based on the detection results of the group of sensors 41, 42, 43.

### <<< Press Device 20 >>>

As shown in Fig. 4A, the press device 20 includes, for example, a male die 21a that can be driven to ascend or descend up and down in the vertical direction, and a female die 21b arranged below the male die 21a in opposition thereto. A substantially horizontal pass line PL is set between the male and female dies 21a, 21b with respect to the up and down direction as the travel path for the belt-like work 1. Accordingly, a section targeted for processing ("processing target section") of the belt-like work 1 is carried in the horizontal direction along the pass line PL, and then, while the carrying is being intermittently halted, the male die 21a descends toward the female die 21b and sandwiches and presses the processing target section of the belt-like work 1, to thus apply press-working. When the male die 21a ascends and the pressing motion is finished, the carrying motion of the belt-like work 1 is resumed, and the press device 20 stays on standby until the next processing target section of the belt-like work 1 moves below the male die 21a and halts. Note that in this example, a hydraulic cylinder (not shown) is employed as a drive source of the ascending and descending motion of the male die 21a, but the invention is not limited thereto.

### <<< Intermittent Carry Device 30 >>>

As shown in Figs. 4A and 4B, the intermittent carry device 30 includes: groups of pass-line rolls 31, 32 serving as the plurality of carry rolls that form the pass line PL for the belt-like work 1 with respect to the press device 20; an entering-side buffer mechanism 34a that is provided within the pass line PL on the upstream side of the press device 20 and that can accumulate the belt-like work 1 in a state strung like a loop that is convex downward in the vertical direction; an exit-side buffer mechanism 34b that is provided within the pass line PL on the downstream side of the press device 20 and that can accumulate the press-worked belt-like work 1 in a state strung like a loop that is convex downward in the vertical direction; a seesaw member 34 serving as an interlocking section for moving the entering-side buffer mechanism 34a and the exit-side buffer mechanism 34b in an interlocked manner; and a tension-variation-suppressing mechanism 37 for suppressing a variation in tension of the belt-like work 1.

As shown in Fig. 4A, the groups of pass-line rolls 31, 32 mainly consist, for example, of a pair of pass-line rolls 31a, 31b arranged at the same height and on the upstream side of the press device 20, and a pair of pass-line rolls 32a, 32b arranged at the same height and on the downstream side of the press device 20. The belt-like work 1 is wrapped around and supported by the pass-line rolls 31a, 31b, 32a, 32b, and thereby the pass line PL for the belt-like work 1 is formed stretching across the press device 20 front-to-rear in the horizontal direction.

The pass-line roll 31b immediately upstream of the press device 20 (also referred to below as a drive roll 31b) is directly connected to a drive source such as a servomotor, and only this pass-line roll 31b is configured as a drive roll that is driven to rotate. Accordingly, the carry state of the belt-like work 1 in the press device 20 is controlled by controlling the rotational speed of the drive roll 31b (which corresponds to the "intermittent sending section" and the "second drive roll"). For example, when the drive roll 31b rotates, the belt-like work 1 in the press device 20 is carried, and on the other hand, when the drive roll 31b halts, the carrying of the belt-like work 1 in the press device 20 also halts. With the present processing apparatus 10, since the above-mentioned press-working is performed under this physical carry-halt state, the press-working can be applied accurately at a target position on the belt-like work 1.

Note that a pressing roll 31c that followingly rotates about a rotation axis parallel to that of the drive roll 31b is pressed against the outer circumferential surface of the drive roll 31b at a predetermined pressing pressure. Being sandwiched between the drive roll 31b and the pressing roll 31c, the belt-like work 1 is carried without causing any relative slippage with the drive roll 31b. Thus, the responsiveness of the carrying motion of the belt-like work 1 by the drive roll 31b is improved, and as a result, the halt position accuracy of the belt-like work 1 is improved.

As shown in Fig. 4A, the entering-side buffer mechanism 34a and the exit-side buffer mechanism 34b are a pair of rolls 34a, 34b supported in a followingly rotatable manner on respective front and rear ends of the seesaw member 34 (which correspond respectively to the "first moving element" and the "second moving element"). These rolls 34a, 34b are equal in diameter and weight. The seesaw member 34, with these rolls 34a, 34b mounted, is in balance in such a manner that it can rotate back and forth about the oscillation central shaft 34c that is located in the middle of the rolls 34a, 34b.

The rolls 34a, 34b are respectively located at a position between the pair of pass-line rolls 31a, 31b and at a position between the pair of pass-line rolls 32a, 32b. Accordingly, the rolls 34a, 34b respectively have a portion 1a of the belt-like work 1 passed over between the pair of pass-line rolls 31a, 31b and a portion 1b of the belt-like work 1 passed over between the pair of pass-line rolls 32a, 32b wrapped around the respective rolls 34a, 34b from below.

Therefore, when the seesaw member 34 oscillates, the amount of the loop (the accumulation amount) of the belt-like work 1 formed by the descending roll 34b (34a) increases, thereby accumulating the belt-like work 1; on the other hand, the amount of the loop (the accumulation amount) of the belt-like work 1 formed by the ascending roll 34a (34b) decreases, thereby sending out the belt-like work 1. That is, the roll 34a of the entering-side buffer mechanism and the roll 34b of the exit-side buffer mechanism always move in an opposite-motion relationship with one another. As a result, the total amount of the length of the loops 1a, 1b formed by these rolls 34a, 34b is always kept constant, and thus, it is possible to reliably achieve synchronization between the entering-side carry speed Vin and the exit-side carry speed Vout of the processing apparatus 10 while keeping the tension of the belt-like work 1 almost constant.

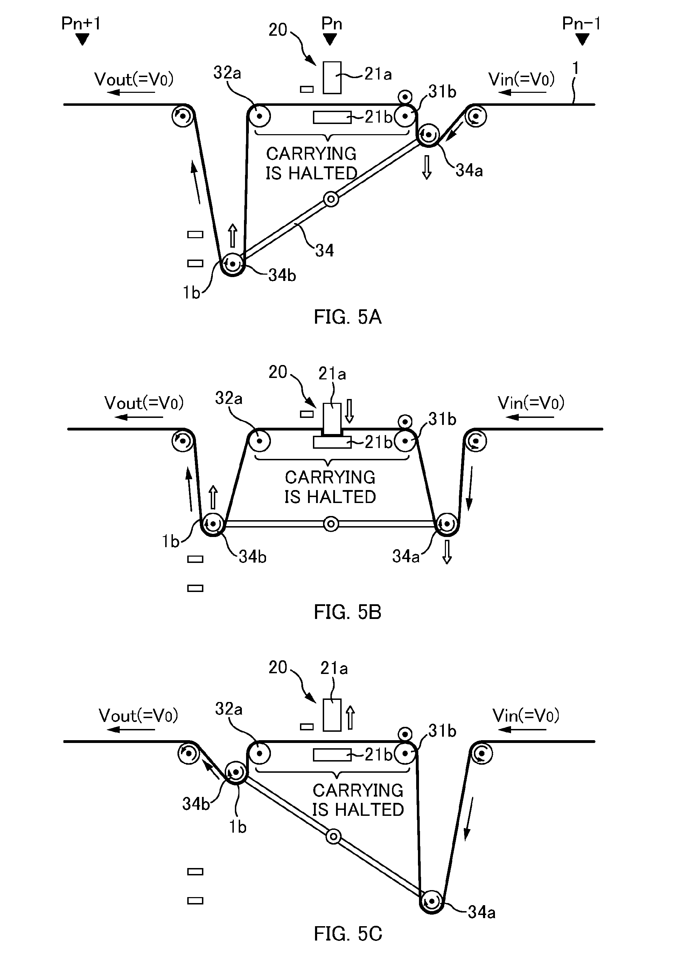

This is described in detail. First, it is assumed that the initial state is a state where a loop 1b is greatly accumulated on the roll 34b of the exit-side buffer mechanism whereas there is almost no loop 1a on the roll 34a of the entering-side buffer mechanism, as shown with the solid lines in Fig. 4A. Even if, in this state, the rotation of the drive roll 31b is halted by the servomotor in order to intermittently halt the carrying of the belt-like work 1 in the press device 20, it is still necessary to send out the belt-like work 1 to the downstream processing position Pn+1 at the exit-side carry speed Vout, regardless of the work being halted. In this case, the roll 34b of the exit-side buffer mechanism (also referred to below as an "exit-side roll 34b") is raised upward owing to the tension of the belt-like work 1 etc., and thus, the belt-like work 1 is sent out from the loop 1b of the exit-side buffer mechanism 34b, as shown in Figs. 5A to 5C. In this way, it is possible to send out the belt-like work 1 at a carry speed Vout equal to the reference speed V0, regardless of the halt of the drive roll 31b.

On the other hand, at this time, the roll 34a of the entering-side buffer mechanism (also referred to below as an "entering-side roll 34a") which is at its upper-limit position descends and accumulates the belt-like work 1, which is carried from the upstream processing position Pn-1, into a downwardly-convex loop shape while pulling the work downward, as shown in Figs. 5A to 5C. Note here that the entering-side roll 34a is also provided on the seesaw member 34, like the exit-side roll 34b. Therefore, the descending motion of the entering-side roll 34a takes place simultaneously and in parallel with the above-mentioned ascending motion of the exit-side roll 34b, as an opposite motion thereof, without any delay in motion. That is, the entering-side roll 34a descends at the same speed as the ascending speed of the exit-side roll 34b, and the descending amount thereof is the same as the ascending amount of the roll 34b. Thus, the amount of the belt-like work 1 accumulated by the roll 34a of the entering-side buffer mechanism becomes equal to the amount of the belt-like work 1 sent out from the roll 34b of the exit-side buffer mechanism. As a result, it is possible to make the entering-side carry speed Vin and the exit-side carry speed Vout substantially equal; in other words, it is possible to reliably achieve synchronization between the entering-side carry speed Vin and the exit-side carry speed Vout.

Note that, when this intermittently-halted state is cancelled, the rotation of the drive roll 31b is resumed, and thus, the belt-like work 1 in the press device 20 is carried until the next intermittent-halt position is reached. During this carrying, however, it is necessary to return the seesaw member 34 from the state shown in Fig. 5C to the initial state shown in Fig. 5A in preparation for the next intermittent halt; this return to the initial state is achieved by appropriately setting the carry speed V of the belt-like work 1 in the press device 20 for the intermittent carrying.

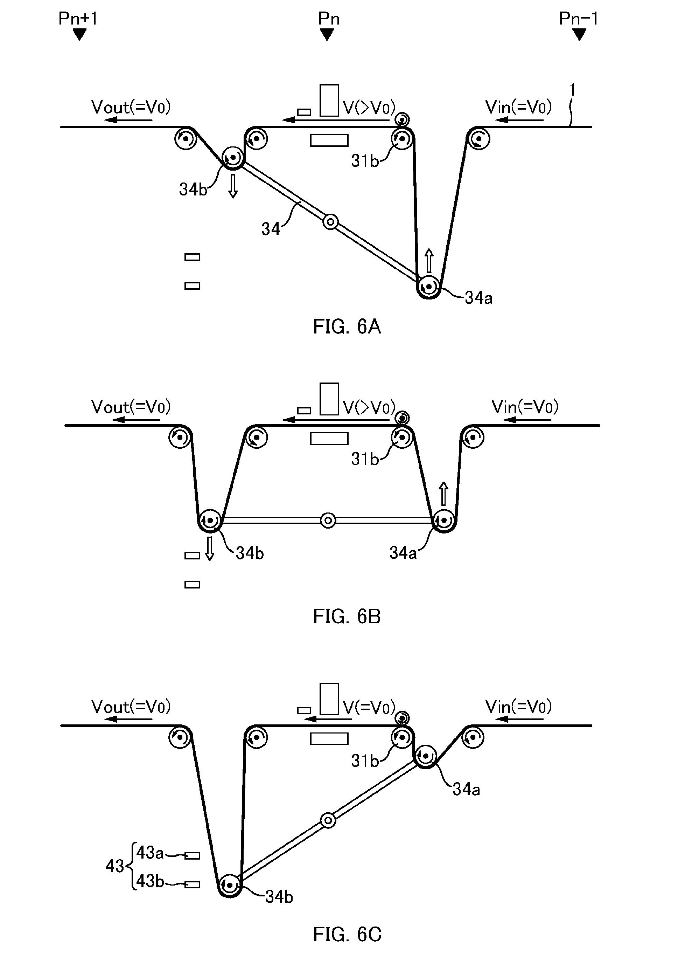

More specifically, when the intermittent halt is released, the drive roll 31b starts to rotate again as shown in Fig. 6A. Here, the rotation speed V thereof at this time is set faster than the entering-side carry speed Vin and the exit-side carry speed Vout. Accordingly, as shown in Figs. 6A to 6C, the belt-like work 1 in the press device 20 is carried at a speed V faster than the entering-side carry speed Vin and the exit-side carry speed Vout, and thus, the amount of belt-like work 1 sent out from the entering-side roll 34a becomes larger than the amount of belt-like work 1 supplied to the entering-side roll 34a at the entering-side carry speed Vin, thereby making the amount of work at the entering-side roll run short. As a result, the amount of the loop at the entering-side roll 34a decreases, and the entering-side roll 34a ascends from its lower-limit position to its upper-limit position. On the other hand, the belt-like work 1 is sent out from the exit-side roll 34b, which is at its upper-limit position, at the carry speed Vout. However, since the belt-like work 1 is supplied to the exit-side roll 34b at a speed V faster than the carry speed Vout, the belt-like work 1 becomes oversupplied. As a result, the amount of the loop at the exit-side roll 34b increases, and the exit-side roll 34b descends from its upper-limit position to its lower-limit position. In other words, the seesaw member 34 carries out a seesawing motion in which the entering-side roll 34a ascends and the exit-side roll 34b descends, and in this way, the seesaw member 34 returns to the above-described initial state.

The tension-variation-suppressing mechanism 37 is provided with the aim of suppressing a variation in tension of the belt-like work 1 due to the above-described seesawing motion etc. More specifically, in this press device 20, as the processing interval becomes short and processing comes to be performed at a short pitch, the oscillation cycle of the seesaw member 34 also becomes short. Accordingly, the influence on the belt-like work 1 of inertia relating to the oscillating motion of the seesaw member 34 as well as inertia relating to the rotating motion of the followingly-rotating pass-line rolls 31a, 32a, 32b becomes too large to neglect, thus causing a variation in tension of the belt-like work 1.

For example, as shown in Figs. 5A and 5B, when the belt-like work 1 is halted, the exit-side roll 34b of the seesaw member 34 is pulled up by the belt-like work 1 and starts to ascend. However, at this time, inertia of the oscillating motion of the seesaw member 34 acts on the belt-like work 1, and this causes a variation in tension of the belt-like work 1. Also, when the belt-like work 1 is halted, the pass-line roll 32a having been rotating following the belt-like work 1 is halted (see Fig. 5A). Therefore, inertia of the rotating motion of the pass-line roll 32a acts on the belt-like work 1, and this also causes a variation in tension of the belt-like work 1. Such a variation in tension may deteriorate the halt position accuracy of the belt-like work 1 and may thus cause the position for processing the belt-like work 1 to deviate from the target position.

In view of the above, the present processing apparatus 10 has a tension-variation-suppressing mechanism 37 for receiving inertia relating to the oscillating motion etc. of the seesaw member 34 and inertia relating to the rotating motion of the pass-line rolls 31a, 32a, 32b in place of the belt-like work 1, as shown in Fig. 4B.

The tension-variation-suppressing mechanism 37 is a so-called wrap-around transmission system and employs, as its main element, an endless belt 37a (corresponding to the "endless annular element") that is circulatingly driven while traveling in parallel with the belt-like work 1 adjacent thereto in the width direction of the belt-like work 1 (the direction passing through the paper face of Fig. 4B). More specifically, the endless belt 37a is wrapped around the pass-line roll 31a, the entering-side roll 34a of the seesaw member 34, the drive roll 31b, the pass-line roll 32a, the exit-side roll 34b of the seesaw member 34, and the pass-line roll 32b in this order along the pass line PL, just like the belt-like work 1. Further, the endless belt 37a is also wrapped around driven rolls 37b, 37c, 37d located below the above-mentioned rolls, thus forming a circulating track of the endless belt 37a.

The endless belt 37a is subjected to the same travel motion and accumulation motion as those of the belt-like work 1 within a range where the endless belt 37a lies along the belt-like work 1. In this way, the endless belt 37a receives, in place of the belt-like work 1, the above-mentioned inertia that may act on the belt-like work 1 at arbitrary timings to thereby suppress a variation in tension of the belt-like work 1.

A configuration example for achieving the above is as follows. First, a drive roll 37e (corresponding to the "first drive roll") that is driven to rotate by employing a servomotor as its drive source is disposed within the circulating track of the endless belt 37a in order to subject the endless belt 37a to the same travel motion as the belt-like work 1. The endless belt 37a is wrapped around the drive roll 37a with a predetermined wrap-around angle. The drive roll 37a rotates at the same peripheral speed V0 as the entering-side carry speed Vin or the exit-side carry speed Vout of the belt-like work 1, and thus the endless belt 37a circulates at the same reference speed V0 as the entering-side carry speed Vin or the exit-side carry speed Vout of the belt-like work 1. Note that the circulating motion of the endless belt 37a also rotates the rolls 31a, 34a, 32a, 34b, 32b around which the endless belt 37a is wrapped.

The endless belt 37a is also wrapped around the drive roll 31b that controls carrying of the belt-like work 1 in order to subject the endless belt 37a to the same accumulation motion as the belt-like work 1. In this way, the drive roll 31b can intermittently halt, and also release the intermittent halt of, the endless belt 37a circulating at the reference speed V0 at the same timing as the belt-like work 1. Thus, the endless belt 37a is subjected to the same accumulation motion as the belt-like work 1 at the entering-side roll 34a and the exit-side roll 34b of the seesaw member 34.

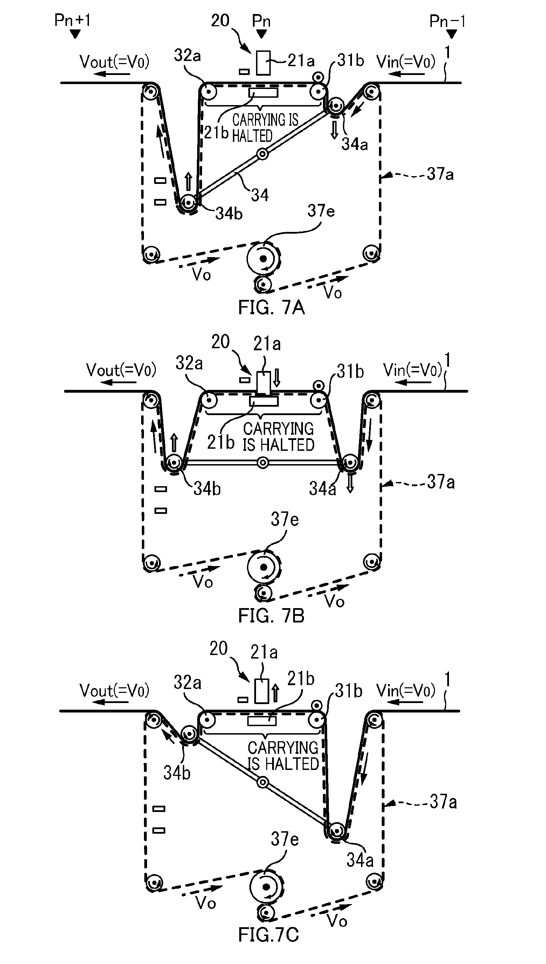

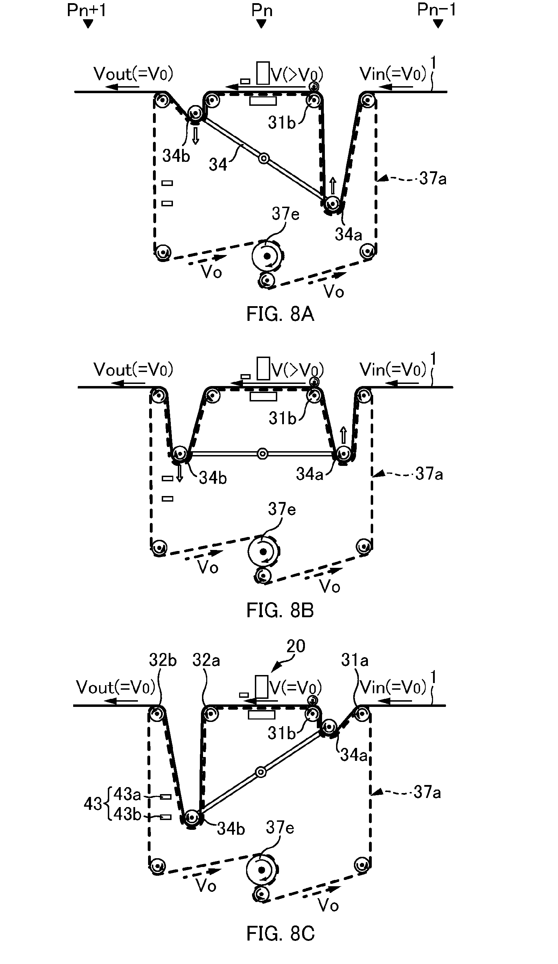

For example, as shown in Fig. 8C, the endless belt 1 travels at the same carry speed V0 as the belt-like work 1 throughout the entire track of the belt while the belt-like work 1 is being carried by the drive roll 31b. However, as shown in Fig. 7A, when the drive roll 31b is intermittently halted, the endless belt 37a located between the drive roll 31b and the carry roll 32a is also intermittently halted by the drive roll 31b, just like the belt-like work 1 located between the drive roll 31b and the carry roll 32a. Also, during this time, the amount of loop of the endless belt 37a on the entering side increases like the belt-like work 1 along with the increase in the amount of loop of the belt-like work 1 on the entering side due to the oscillating motion of the seesaw member 34, and the amount of loop of the endless belt 37a on the exit side decreases along with the decrease in the amount of loop of the belt-like work 1 on the exit side, as shown in Figs. 7B and 7C. On the other hand, when the intermittent halt is released, the endless belt 37a is also sent by the drive roll 31b at the same speed V as the belt-like work 1, as shown in Fig. 8A. Then, as shown in Figs. 8B and 8C, the amount of loop of the endless belt 37a on the entering side decreases like the belt-like work 1 along with the decrease in the loop of the belt-like work 1 on the entering side due to the oscillating motion of the seesaw member 34, and the amount of loop of the endless belt 37a on the exit side increases along with the increase in the amount of loop of the belt-like work 1 on the exit side, thus finally returning the endless belt 37a to its initial state shown in Fig. 8C.

### <<< Group of Sensors 41, 42, 43 >>>

As shown in Fig. 4A, the group of sensors 41, 42, 43 includes a pressing-motion monitoring sensor 41 for monitoring the pressing motion of the press device 20, a processing-target-section monitoring sensor 42 for monitoring the position of the processing target section on the belt-like work 1, and an oscillating-motion monitoring sensor 43 for monitoring the oscillating motion of the seesaw member 34.

The pressing-motion monitoring sensor 41 is, for example, a proximity switch provided at the upper-limit position of the male die 21a and outputs a detection signal every time the male die 21a reaches the upper-limit position.

The processing-target-section monitoring sensor 42 is a sensor that is arranged immediately upstream of the press device 20 and that outputs a detection signal every time the sensor detects a mark indicating a processing location (referred to below as a "processing-location mark") formed on the belt-like work 1 at a predetermined pitch. An example thereof includes a photoelectric tube that outputs a signal having an intensity corresponding to the amount of light received.

The oscillating-motion monitoring sensor 43 is, for example, a proximity switch provided near the lower-limit position of the exit-side roll 34b of the seesaw member 34, and the proximity switch outputs a detection signal when the exit-side roll 34b reaches the lower-limit position. Note here that two positions--an upper lower-limit position, and a lower lower-limit position situated slightly below the upper lower-limit position--are set as the lower-limit position, and proximity switches 43a, 43b are respectively arranged at those positions.

### <<< Controller >>>

The controller is a suitable sequencer and/or computer, and controls the various drive sources relating to the processing apparatus 10 based on the detection results output from the above-described group of sensors 41, 42, 43. More specifically, the controller controls the hydraulic cylinder that drives the male die 21a of the press device 20 so that it ascends and descends, and controls the rotational speed of the servomotor, which serves as the drive source of the drive roll 31b. Further, the controller controls the rotational speed of the servomotor of the drive roll 37a in such a manner that the peripheral speed of the drive roll 37a of the tension-variation-suppressing mechanism 37 becomes the reference speed V0, which is the same as the entering-side carry speed Vin or the exit-side carry speed Vout of the belt-like work 1.

### <<< Operation Example of Processing Apparatus 10 >>>

With the processing apparatus 10 configured as above, rotating the drive roll 31b as described below allows the belt-like work 1 to be intermittently carried in the press device 20 while suppressing variation in tension and allows press-working to be applied to the belt-like work 1 at an appropriate pitch, while maintaining the carry speed Vin, Vout of the belt-like work 1 at the respective processing positions Pn-1, Pn+1 upstream and downstream of the press device 20 at the normal reference speed V0.

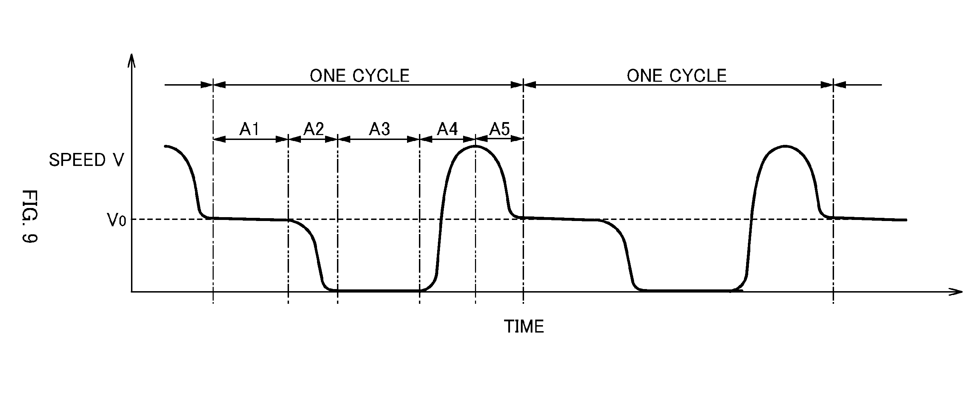

Fig. 9 is a chart showing the rotation speed V of the drive roll 31b. The horizontal axis indicates time, and the vertical axis indicates speed (meters/second). Note that, since the carrying of the belt-like work 1 in the press device 20 is controlled by the drive roll 31b, the vertical axis of Fig. 9 also indicates the carry speed V of the belt-like work 1 that is carried through the press device 20.

First, in the initial state, it is assumed that the entering-side roll 34a and the exit-side roll 34b of the seesaw member 34 are respectively located at the upper-limit position and the lower-limit position as shown in Fig. 8C, and the belt-like work 1 in the press device 20 is being carried by the drive roll 31b at the reference speed V0 equal to the entering-side carry speed Vin and the exit-side carry speed Vout.

When a detection signal indicating detection of a processing-location mark on the belt-like work 1 is transmitted from the processing-target-section monitoring sensor 42 during the normal range A1 of Fig. 9 in which carrying is performed in the initial state, the controller halts the rotation of the drive roll 31b according to a predetermined deceleration pattern as shown in the deceleration range A2 of Fig. 9, to thus intermittently halt the carrying of the belt-like work 1 in the press device 20.

During this intermittent halt A3, the controller makes the press device 20 perform its pressing motion (Figs. 7A and 7B).

Note that during this intermittent halt A3, the seesaw member 34 performs its oscillating motion from the initial state (Fig. 7A) to the opposite state (Fig. 7C) as described above (that is, the entering-side roll 34a descends while the exit-side roll 34b ascends). Thus, it is possible to take in the belt-like work 1 sent in from the upstream processing position Pn-1 at the reference speed V0 in its stretched-out state and to send out the belt-like work 1 to the downstream processing position Pn+1 at the reference speed V0. Therefore, the carrying state at the respective upstream and downstream processing positions Pn-1, Pn+1 is not interrupted whatsoever by the intermittent halt. Further, this oscillating motion is basically achieved by the exit-side roll 34b of the seesaw member 34 being raised due to the tension of the belt-like work 1; at this time, the tension of the endless belt 37a also acts in the direction for raising the exit-side roll 34b, and this works to suppress a variation in tension of the belt-like work 1.

When receiving a signal indicating completion of the pressing motion from the pressing-motion monitoring sensor 41 after a while, the controller resumes the rotation of the drive roll 31b. At this time, however, as shown in the acceleration range A4 of Fig. 9, the controller increases the rotation speed according to a predetermined acceleration pattern up to a speed faster than the reference speed V0 in order to make the speed eventually faster than the entering-side carry speed Vin and the exit-side carry speed Vout, to thus return the seesaw member 34 in the state shown in Fig. 8A back to the initial state shown in Fig. 8C and prepare for the intermittent halt for the next pressing motion. Note that the oscillating motion of the seesaw member 34 at this time is also basically achieved by the entering-side roll 34a of the seesaw member 34 being raised due to the tension of the belt-like work 1; at this time, the tension of the endless belt 37a also acts in the direction for raising the entering-side roll 34a, and this works to suppress a variation in tension of the belt-like work 1.

Incidentally, the fact that the seesaw member 34 has returned to its initial state (Fig. 8C) is detected by the oscillating-motion monitoring sensor 43. This is described in detail. Immediately before returning to the initial state, the exit-side roll 34b first passes by the position of the sensor 43a at the upper lower-limit position, and therefore, the sensor 43a at the upper lower-limit position sends a detection signal. Then, the controller starts to decelerate the drive roll 31b, as shown in the deceleration range A5 of Fig. 9. Then, when the exit-side roll 34b reaches the lower lower-limit position and the sensor 43b at the lower lower-limit position sends a detection signal, the controller sets the rotation speed of the drive roll 31b to the reference speed V0, which completes a single processing cycle.

After this, the above-described processing cycle is repeated every time the processing-target-section monitoring sensor 42 detects a processing-location mark on the belt-like work 1.

### === SECOND EMBODIMENT ===

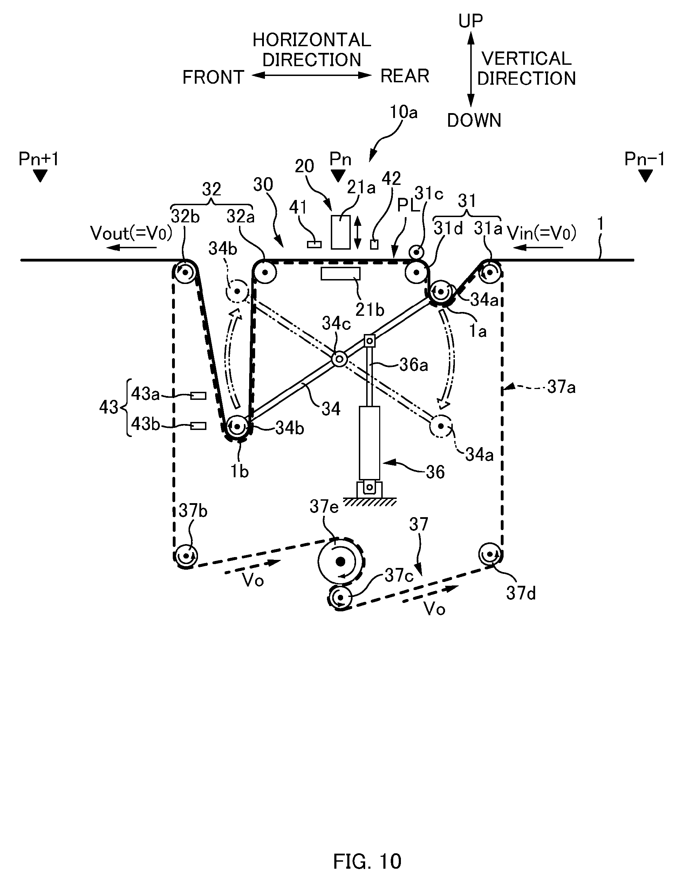

Fig. 10 is a side view of a processing apparatus 10a according to a second embodiment. In the above-described first embodiment, the drive roll 31b controls the carrying state of the belt-like work 1 in the press device 20. In the present second embodiment, the carrying state is controlled by a brake-equipped non-drive roll 31d provided in place of the drive roll 31b and an oscillation drive device 36 for driving the seesaw member 34 to oscillate. In other words, the brake-equipped non-drive roll 31d and the oscillation drive device 36 correspond to the "intermittent sending section". The features other than the above are almost the same as those in the first embodiment, and for example, the tension-variation-suppressing mechanism 37 is provided likewise.

The brake-equipped non-drive roll 31d includes a non-drive roll 31d installed at the same position as the drive roll 31b in place thereof, and a brake mechanism (not shown) of, for example, the drum-type or the disk-type for braking the rotation of the non-drive roll 31d. Accordingly, when the brake mechanism is not in operation, the non-drive roll 31d rotates along with the carrying of the belt-like work 1 and the endless belt 37a which are in contact with the roll, whereas when the brake mechanism is in operation, not only is the roll itself halted, but the belt-like work 1 and the endless belt 37a in contact therewith are also halted.

The oscillation drive device 36 is, for example, an air cylinder, and the tip end of its piston 36a is connected to the seesaw member 34. Thus, by supplying compressed air (pressurized air) from a predetermined compressed-air source to a cylinder chamber in the air cylinder via a diverter valve such as a solenoid valve, it is possible to oscillate the seesaw member 34 up and down via the ascending and descending motion of the piston 36a.

The non-drive roll 31d and the oscillation drive device 36 are controlled by the above-described controller, and thus, the belt-like work 1 is intermittently carried in the press device 20 as follows.

As in the above example, explanation is made based on the assumption that the processing apparatus 10a is in the initial state shown in Fig. 8C. More specifically, the entering-side roll 34a and the exit-side roll 34b of the seesaw member 34 are respectively located at the upper-limit position and the lower-limit position as shown by the solid lines in Fig. 10, and the belt-like work 1 in the press device 20 is pulled by the downstream processing position Pn+1 and thus carried at the reference speed V0 equal to the entering-side carry speed Vin and the exit-side carry speed Vout.

When a detection signal indicating detection of a processing-location mark is transmitted from the processing-target-section monitoring sensor 42 while the work is being carried in the initial state, the controller activates the brake mechanism to stop the rotation of the non-drive roll 31d, to thus intermittently halt the belt-like work 1 and the endless belt 37a in the press device 20.

Then, during this intermittent halt, the controller makes the press device 20 perform its pressing motion.

Note that during this intermittent halt, the cylinder chamber of the air cylinder 36 is cut off from the compressed-air source by the diverter valve and opened to the atmosphere, and thus, the seesaw member 34 is brought to a state where it can freely oscillate owing to even the slightest load. Thus, the exit-side roll 34b of the seesaw member 34 is raised by the tension of the belt-like work 1 as well as the tension of the endless belt 37a and ascends, whereas the entering-side roll 34a performs the opposite motion and descends. In other words, the seesaw member 34 performs its oscillating motion from the initial state shown with the solid lines in Fig. 10 to the opposite state shown with the chain double-dashed lines. Thus, the seesaw member 34 can take in the belt-like work 1 sent in from the upstream processing position Pn-1 at the entering-side carry speed Vin in its stretched-out state and send out the belt-like work 1 to the downstream processing position Pn+1 at the exit-side carry speed Vout. Therefore, the carrying state at the respective upstream and downstream processing positions Pn-1, Pn+1 is not interrupted whatsoever by the intermittent halt.

Then, when receiving a signal indicating completion of the pressing motion from the pressing-motion monitoring sensor 41, the controller releases the brake on the non-drive roll 31d. Then, the non-drive roll 31d comes to rotate along with the belt-like work 1 carried by being pulled by the downstream processing position Pn+1 and the endless belt 37a. During this carrying, however, it is necessary to return the seesaw member 34 to the initial state shown with the solid lines (i.e., to the state in which the entering-side roll 34a is at the upper-limit position and the exit-side roll 34b is at the lower-limit position) in preparation for the next intermittent halt. To do so, the controller switches the diverter valve and supplies the compressed air from the compressed-air source to the cylinder chamber of the air cylinder 36, thereby extending the piston 36a of the air cylinder 36 and oscillating the seesaw member 34, i.e., raising the entering-side roll 34a and lowering the exit-side roll 34b.

Note that the fact that the seesaw member has returned to its initial state is detected by the oscillating-motion monitoring sensor 43. This is described in detail. When the exit-side roll 34b reaches the lower lower-limit position and the sensor 43b at the lower lower-limit position sends a detection signal, the controller halts the extending motion of the air cylinder 36, which completes a single processing cycle.

Incidentally, the above-described second embodiment uses a brake-equipped non-drive roll 31d for intermittently halting the belt-like work 1 and releasing the same. It is instead possible to use a simple non-drive roll (driven roll) having no brake mechanism. In this case, however, a separate mechanism for restricting and halting the belt-like work 1 and the endless belt 37a becomes necessary. An example thereof may include a nip mechanism that is arranged immediately downstream of the non-drive roll and that includes a pair of upper and lower nipping members provided so that they can nip the belt-like work 1 and the endless belt 37a from above and below. When halting the carrying of the belt-like work 1, the pair of upper and lower nipping members moves toward one another and nips the belt-like work 1 and the endless belt 37a simultaneously, to thereby restrict the downstream movement of the belt-like work 1 and the endless belt 37a. On the other hand, when resuming the carrying of the belt-like work 1, the nipping members move away from one another to thus cancel the nipped state of the belt-like work 1 and the endless belt 37a.

### === MODIFIED EXAMPLE OF SECOND EMBODIMENT ===

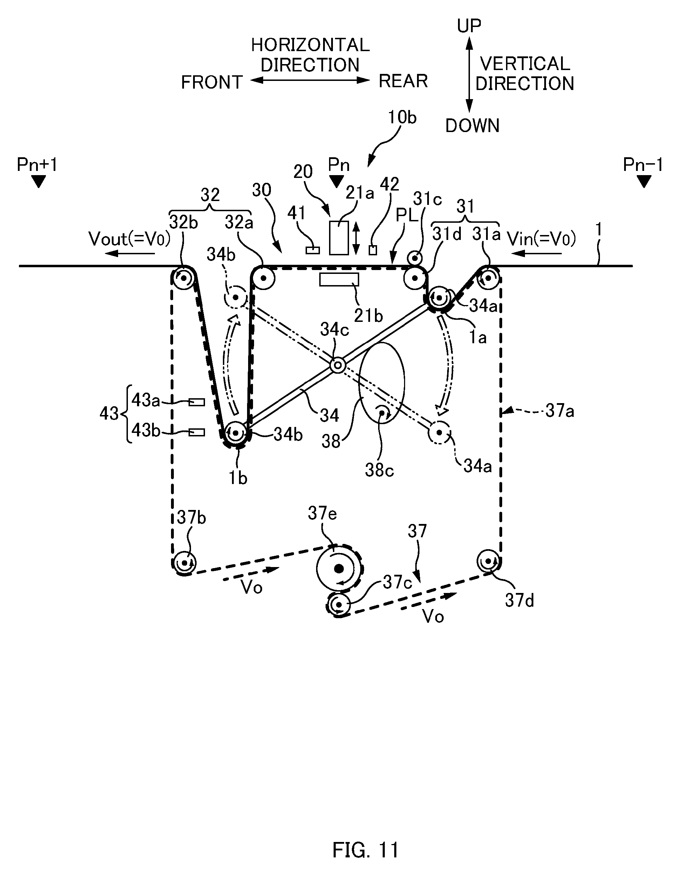

Fig. 11 is a side view of a processing apparatus 10b according to a modified example of the second embodiment. The second embodiment uses an air cylinder as the oscillation drive device 36 of the seesaw member 34, but the present modified example uses a cam mechanism instead. The features other than this are almost the same as those in the second embodiment, and for example, a brake-equipped non-drive roll 31d is disposed in place of the drive roll 31b, like the above-described second embodiment.

The oscillation drive device 38 employs, as its main element, a substantially-oval disk-like plate cam 38 that is rotatable about a predetermined axis 38c, for example. The plate cam 38 is arranged in such a manner that its outer circumferential surface serving as a cam surface comes into contact with the lower surface of the seesaw member 34 at a position more to the entering side than the oscillation central shaft 34c of the seesaw member 34. Accordingly, by rotating the plate cam 38 about the axis 38c with a servomotor as its drive source, it is possible to oscillate the seesaw member 34 up and down.

The plate cam 38 and the brake-equipped non-drive roll 31d are controlled by the above-described controller, and in this way, the belt-like work 1 is intermittently carried in the press device 20 as follows.

As in the above example, explanation is made based on the assumption that the processing apparatus 10b is in the initial state shown in Fig. 8C. More specifically, as shown with the solid lines in Fig. 11, the seesaw member 34 is supported from below by the plate cam 38 in such a manner that it cannot oscillate, the plate cam 38 being halted at its top dead center. Accordingly, the entering-side roll 34a and the exit-side roll 34b of the seesaw member 34 are respectively located at the upper-limit position and the lower-limit position. Further, the belt-like work 1 is pulled by the downstream processing position Pn+1 and thus carried through the press device 20 at the reference speed V0 equal to the entering-side carry speed Vin and the exit-side carry speed Vout.

When a detection signal indicating detection of a processing-location mark is transmitted from the processing-target-section monitoring sensor 42 while the work is being carried in the initial state, the controller activates the brake mechanism to stop the rotation of the non-drive roll 31d, to thus intermittently halt the belt-like work 1 and the endless belt 37a in the press device 20.

Then, during this intermittent halt, the controller makes the press device 20 perform its pressing motion.

Note that at the time of this intermittent halt, the plate cam 38 starts to rotate and is brought to a state where it does not support the seesaw member 34, and thus, the seesaw member 34 is brought to a state where it can freely oscillate owing to even the slightest load. Thus, the exit-side roll 34b of the seesaw member 34 is raised by the tension of the belt-like work 1 as well as the tension of the endless belt 37a and ascends, whereas the entering-side roll 34a performs the opposite motion and descends. In other words, the seesaw member 34 performs its oscillating motion from the initial state shown with the solid lines in Fig. 11 to the opposite state shown with the chain double-dashed lines. Thus, the seesaw member 34 can take in the belt-like work 1 sent in from the upstream processing position Pn-1 at the entering-side carry speed Vin in its stretched-out state and send out the belt-like work 1 to the downstream processing position Pn+1 at the exit-side carry speed Vout. Therefore, the carrying state at the respective upstream and downstream processing positions Pn-1, Pn+1 is not interrupted whatsoever by the intermittent halt.

Then, when receiving a signal indicating completion of the pressing motion from the pressing-motion monitoring sensor 41, the controller releases the brake on the non-drive roll 31d. Then, the non-drive roll 31d comes to rotate along with the belt-like work 1 carried by being pulled by the downstream processing position Pn+1 and the endless belt 37a. During this carrying, however, it is necessary to return the seesaw member 34 to the initial state shown with the solid lines (i.e., to the state in which the entering-side roll 34a is at the upper-limit position and the exit-side roll 34b is at the lower-limit position) in preparation for the next intermittent halt. To do so, the controller rotates the plate cam 38 so that the plate cam comes into contact with the lower surface of the seesaw member 34 to make the seesaw member 34 oscillate, thereby raising the entering-side roll 34a and lowering the exit-side roll 34b.

Note that the fact that the seesaw member has returned to its initial state is detected by the oscillating-motion monitoring sensor 43. This is described in detail. Immediately before returning to the initial state, the exit-side roll 34b passes by the position of the sensor 43a at the upper lower-limit position, and therefore, the sensor 43a at the upper lower-limit position sends a detection signal. Then, the controller first starts to decelerate the rotating motion of the plate cam 38. Then, when the exit-side roll 34b reaches the lower lower-limit position and the sensor 43b at the lower lower-limit position sends a detection signal, the controller halts the rotating motion of the plate cam 38, which completes a single processing cycle.

### === OTHER EMBODIMENTS ===

Although embodiments of the present invention have been described above, the invention is not limited to those embodiments, and modifications such as those described below are possible.

The first embodiment illustrates a drive roll 31b as an intermittent sending section and also illustrates a configuration in which an endless belt 37a is directly wrapped around the drive roll 31b. The invention, however, is not limited thereto whatsoever, as long as the intermittent sending section is configured to intermittently halt the endless belt 37a in the same way as the belt-like work 1.

For example, the endless belt 37a does not have to be directly wrapped around the drive roll 31b; simply transmitting the rotational force from a drive source of the drive roll 31b via a suitable wrap-around transmission system to intermittently halt the endless belt 37a at the same timing as the belt-like work 1 will do.

In the foregoing embodiments, the range where the endless belt 37a lies along the pass line PL (travel path) of the belt-like work 1 is set to the entire range from the pass-line roll 31a to the pass-line roll 32b, as shown in Fig. 4B or Fig. 8C. The range, however, is not limited thereto whatsoever, and the endless belt may be set so that it does not lie along a portion of the pass line PL.

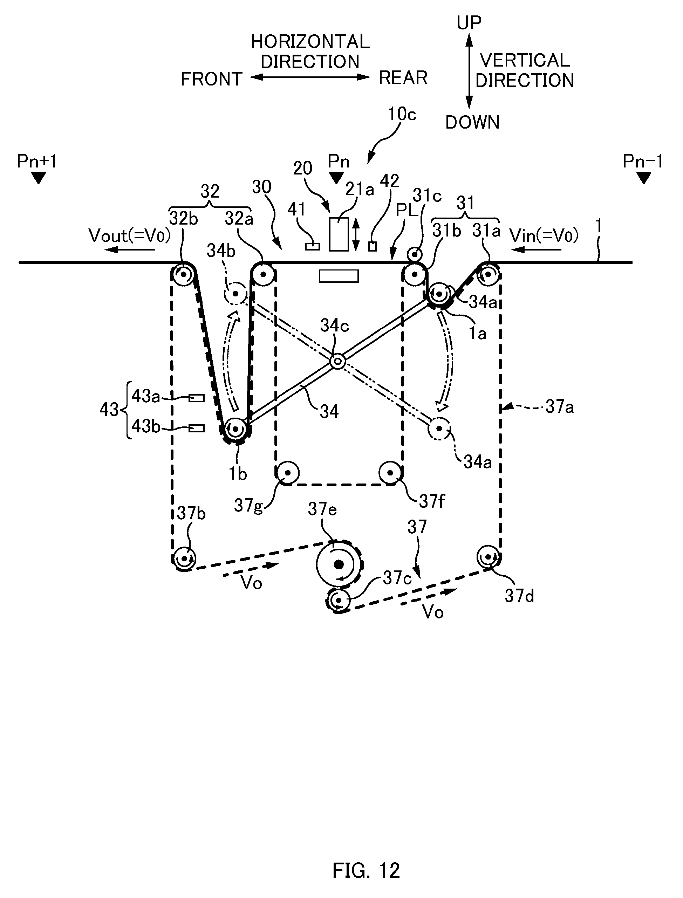

For example, driven rolls 37f, 37g such as those shown in Fig. 12 may be disposed and the endless belt 37a may also be wrapped around these rolls 37f, 37g so that the endless belt 37a does not lie along the belt-like work 1 at the position of the press device 20--i.e., between the drive roll 31b and the pass-line roll 32a.

The foregoing embodiments illustrate a seesaw member 34 as an interlocking section that makes the entering-side roll 34a of the entering-side buffer mechanism and the exit-side roll 34b of the exit-side buffer mechanism carry out opposite motions in an interlocked manner. The interlocking section, however, is not limited thereto whatsoever, as long as it can make the two members carry out opposite motions in an interlocked manner. That is, the two rolls do not have to be connected together by the seesaw member 34.

For example, the entering-side roll 34a and the exit-side roll 34b may be configured so that they are reciprocatably guided in the up and down direction respectively, for example, by suitable guide rails and that the entering-side roll 34a and the exit-side roll 34b can ascend or descend up and down by a drive source such as an air cylinder. Further, this air cylinder may be controlled by a suitable controller such as a computer in such a manner that the entering-side roll 34a and the exit-side roll 34b perform opposite motions from one another (i.e., move in opposite directions from one another at the same speed).

The foregoing embodiments do not particularly describe the materials etc. for the belt-like work 1. However, any belt-like element having moderate flexibility is applicable, and examples thereof may include nonwoven fabrics, woven fabrics, sheets, and film-like elements. Materials therefor may include resins, such as synthetic resins, and pulp.

The foregoing embodiments give press-working as an example of processing applied to the belt-like work 1. The invention, however, is not limited thereto whatsoever, and for example, the processing may include embossing for applying projecting-and-depressed patterns through pressing with dies, and sealing for melt-joining the belt-like work 1.

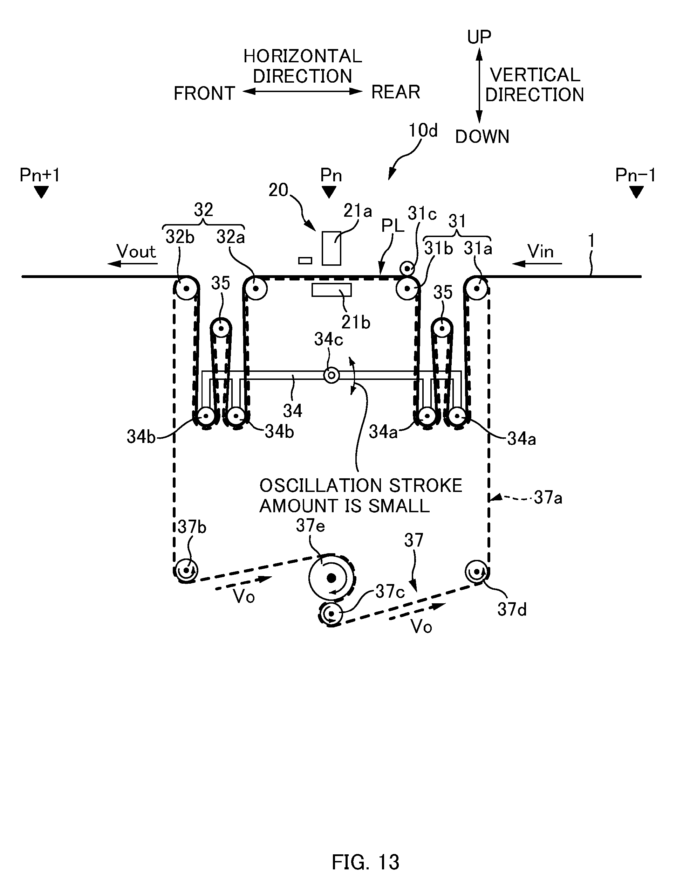

In the foregoing embodiments, one entering-side roll 34a and one exit-side roll 34b are provided on the seesaw member 34 to thus form a single downwardly-convex loop on each roll. It is, however, possible to increase the number of loops to two or more. For example, as shown in Fig. 13, a plurality of loops (two loops in this example) consisting of the belt-like work 1 may be formed by: providing two each of the entering-side rolls 34a and the exit-side rolls 34b on the seesaw member 34; providing respective fixed rolls 35 (rolls that are fixed at a predetermined position so that they do not move) between the entering-side rolls 34a, 34a and between the exit-side rolls 34b, 34b; and wrapping the belt-like work 1 around the rolls in a zigzag form. Note that increasing the number of loops to two or more allows the stroke amount of the oscillating motion of the seesaw member 34 to be reduced compared to a case where there is only one loop, and thus, it is possible to reduce the oscillating motion speed and thereby reduce the impact upon vertically reversing the oscillating motion.

## Claims

1. A processing apparatus comprising:
a work carrying section that carries a continuous belt-like work by wrapping the work around a plurality of carry rolls and forming a travel path for the work; and
a processing section that is provided within the travel path and that performs processing while the work is being intermittently halted;
wherein the work carrying section includes
an entering-side buffer mechanism that is provided within the travel path on an upstream side of the processing section and that can accumulate the work carried from upstream by wrapping the work around a first moving element that is guided to be able to perform a reciprocating movement,
an exit-side buffer mechanism that is provided within the travel path on a downstream side of the processing section and that can accumulate the work having been processed and to be carried downstream by wrapping the work having been processed around a second moving element that is guided to be able to perform a reciprocating movement,
an interlocking section that moves the first moving element and the second moving element in an interlocked manner in such a manner that a motion of the entering-side buffer mechanism for increasing or decreasing an accumulation amount of the work accumulated thereby and a motion of the exit-side buffer mechanism for increasing or decreasing an accumulation amount of the work accumulated thereby are opposite from one another and in such a manner that an amount of change in the accumulation amount of the work of the entering-side buffer mechanism and an amount of change in the accumulation amount of the work of the exit-side buffer mechanism are equal,
an intermittent sending section that comes into contact with the work which has passed through the entering-side buffer mechanism and that repeats a halt motion for intermittently halting the work and a sending motion for sending out the work to the processing section, an endless annular element that is wrapped around the carry rolls, the first moving element, and the second moving element in such a manner that the endless annular element lies along the work within the travel path, and
a first drive roll that, by circulatingly driving the endless annular element, applies a rotational force to the carry rolls and also applies a moving force to the first moving element and the second moving element for achieving the reciprocating movement thereof; and
wherein the first drive roll continuously drives the endless annular element circulatingly at a predetermined speed while the intermittent sending section intermittently halts the endless annular element in synchronization with the halt motion of the work in such a manner that the endless annular element is subjected to the same travel motion and accumulation motion as those of the work within a range where the endless annular element lies along the travel path.

2. A processing apparatus according to claim 1, wherein:
the first moving element of the entering-side buffer mechanism is a roll that rotates followingly;
the second moving element of the exit-side buffer mechanism is a roll that rotates followingly; and
the rolls rotate by receiving the rotational force caused by circulatingly driving the endless annular element.

3. A processing apparatus according to claim 1 or 2, further comprising:
a seesaw member as the interlocking section, the seesaw member oscillating about a predetermined axis;
wherein the first moving element and the second moving element are mounted on the seesaw member; and
wherein the axis is located at a midpoint between a position where the first moving element is mounted and a position where the second moving element is mounted, and an oscillating motion of the seesaw member causes the reciprocating movement of the first moving element and the second moving element.

4. A processing apparatus according to claim 3, wherein:
the seesaw member includes a plurality of the first moving elements for wrapping the work around the first moving elements in a zigzag form, and a plurality of the second moving elements for wrapping the work having been processed around the second moving elements in a zigzag form.

5. A processing apparatus according to any one of claims 1 to 4, wherein:
the predetermined speed of the endless annular element is a speed at a contacting position where the first drive roll comes into contact with the endless annular element; and
the predetermined speed is equal to
a travel speed of the work on a side upstream of the entering-side buffer mechanism or
a travel speed of the work having been processed on a side downstream of the exit-side buffer mechanism.

6. A processing apparatus according to any one of claims 1 to 5, wherein:
the intermittent sending section is a second drive roll that carries the work to the processing section by being driven to rotate while winding the work from the entering-side buffer mechanism around an outer circumferential surface of the roll; and
the endless annular element is also wound around the second drive roll.

7. A processing apparatus according to any one of claims 1 to 6, wherein:
while the halt motion of the work is cancelled, the intermittent sending section performs, in parallel, an increasing motion for increasing the accumulation amount of the exit-side buffer mechanism and a decreasing motion for decreasing the accumulation amount of the entering-side buffer mechanism; and
the increasing motion and the decreasing motion are achieved by making a travel speed of the work between the entering-side buffer mechanism and the exit-side buffer mechanism faster than both
a travel speed of the work on a side upstream of the entering-side buffer mechanism and
a travel speed of the work having been processed on a side downstream of the exit-side buffer mechanism.
